(19)

Europäisches
Patentamt
European
Patent Office
Office européen
des brevets

(11) **EP 3 477 657 A1**

(12) **EUROPEAN PATENT APPLICATION**

(43) Date of publication:
**01.05.2019 Bulletin 2019/18**

(51) Int Cl.:
***G16H 50/70*** *(2018.01)*

(21) Application number: **17198719.1**

(22) Date of filing: **26.10.2017**

<table>
<tr><td>

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Advanced MR Analytics AB
582 22 Linköping (SE)**

</td><td>

(72) Inventors:
- **Borga, Magnus
  582 39 Linköping (SE)**
- **Dahlqvist Leinhard, Olof
  582 74 Linköping (SE)**
- **LINGE, Jennifer
  582 24 Linköping (SE)**

(74) Representative: **AWA Sweden AB
Östra Storgatan 7
553 21 Jönköping (SE)**

</td></tr>
</table>

(54) **EVALUATING AN INDIVIDUAL'S CHARACTERISTICS OF AT LEAST ONE PHENOTYPE VARIABLE**

(57)      The present invention relates to a method (100) of providing a basis for an evaluation of an individual's propensity for a certain health state. The method comprises the steps of acquiring (102) at least two of the following parameter values of the individual's body out of the group: amount of visceral fat, amount of subcutaneous fat, volume of at least one tissue compartment, concentration of fat infiltrated in at least one tissue compartment, concentration of fat infiltrated in at least one organ, and concentration of fat in bone marrow; determining (104) a body composition profile, BCP, for the individual using said at least two acquired parameter values in combination, and comparing (106) the BCP for the individual with parameter values which are based on previously stored BCPs of other individuals.

Fig. 5

Printed by Jouve, 75001 PARIS (FR)

## Description

Technical Field

The present invention relates to a method of providing a basis for an evaluation of an individual's characteristics of at least one phenotype variable, and especially to such method using said individual's body composition parameter values.

Background

An individual's characteristics of a phenotype variable is interesting to evaluate in order to be able to act preventively with regards to said health status, i.e. the possibility or risk for development of a particular disease or syndrome. This could for instance be connected to the individual's propensity for type 2 diabetes, cardiovascular diseases (CVD), high blood pressure, angina, stroke or heart attack.

It is common to use BMI (Body Mass Index), having the individual's length and weight as input parameters, as a measurement of some propensity determinations. It is however an established fact that anthropometric measures, such as BMI, are poor predictors of body fat distribution and associated metabolic risk, particularly at an individual level.

Consequently, a more accurate basis for evaluating an individual's characteristics of phenotype variables is needed.

Summary

It is an object of the present invention to provide an improved method for providing a basis for an evaluation of an individual's characteristics of at least one phenotype variable compared to known methods.

The invention is defined by the appended independent claims, with embodiments being set forth in the appended dependent claims, in the following description and in the drawings.

According to a first aspect of the invention, a method of providing a basis for an evaluation of an individual's characteristics of at least one phenotype variable is provided. The method comprises the steps of acquiring at least two of the following parameter values of the individual's body out of the group: amount of visceral fat, amount of subcutaneous fat, volume of at least one tissue compartment, concentration of fat infiltrated in at least one tissue compartment, concentration of fat infiltrated in at least one organ, and concentration of fat in bone marrow; determining a body composition profile, BCP, for the individual using said at least two acquired parameter values in combination, and comparing the BCP for the individual with parameter values which are based on previously stored BCPs of other individuals.

The characteristics of at least one phenotype variable may be a parametric or non-parametric description of the at least one phenotype variable. The phenotype variable may for instance be HBA1c, blood pressure, age, or health state.

The outcome of the comparison of the method according to the present invention may be that the $BCP_I$ is connected or categorized to previously stored BCP based parameter values. Based on such connection or categorization, an evaluation of the individual's characteristics of at least one phenotype variable may be made based on phenotype variable data connected to said parameter values based on previously stored BCPs. The phenotype variable data may for instance be collected data about the individuals of the BCPs and/or their medical data. The phenotype variable data may provide a mean value and variance of the phenotype variable.

The characteristics of at least one phenotype variable may in one embodiment be provided as a propensity for a certain health state. The invention may thereby relate to a method of providing a basis for an evaluation of an individual's propensity for a certain health state.

The propensity for a certain health state may be a propensity for a certain disease or syndrome. By evaluating an individual's propensity for a certain health state it may be meant a determination of the individual's propensity for a certain health state based on retrospectively collected data. The retrospectively collected data may as an example indicate that a specific amount or share of the individuals of the previously stored BCPs had high blood pressure.

The evaluation of an individual's characteristics of at least one phenotype variable may also be meant to comprise a determination of a risk of a future disease for the individual. For such determination, prospective data for individuals represented in the previously stored BCPs may be used.

Body composition profiling provided by the present invention may allow for a quick and simultaneous assessment of an individual's or group's fat accumulation pattern, fat and muscle distribution, and balance between adipose tissue compartments. Different phenotypes described in a multivariable space may thereby be distinguished. The determined BCP may provide an easy interpreted multivariable representation to highlight the ectopic fat compartments and a quick assessment of a subject's propensity or risk profile by evaluating the characteristics of at least one phenotype variable.

The parameter values may be provided by a previous partial or full body MRI scan of the individual's body. Different methods may be used to extract the parameter values from the resulting image of the MRI scan. The MRI scan may provide a water and fat separated image. The BCP of the individual, the $BCP_I$, may be formed by combining two or more acquired parameter values. The combined parameter values may provide a two or more-dimensional representation constituting the $BCP_I$, which may be visualized in a two or more-dimensional way.

The parameter values may be used as predictors for one or more factors that may affect the individual's characteristics of a phenotype variable.

An amount of visceral fat may provide a parameter value representing the mass or volume of visceral fat present in the individual's body.

An amount of subcutaneous fat may provide a parameter value representing the mass or volume of subcutaneous fat in specific regions of the individual's body, such as the abdominal area or thighs. The abdominal area in the individual's body may be defined as the part of the abdomen from the top of femoral head to the top of vertebrae T9.

A volume of at least one tissue compartment may provide a parameter value representing the volume of a certain tissue compartment, such as a muscle, in the individual's body. The tissue compartment may be predetermined. Such predetermined tissue compartment may be one of the muscles in the group of: the left or the right front thigh muscle, the left or the right back thigh muscle.

A concentration of fat infiltrated in at least one tissue compartment may provide a parameter value representing a percentage or ratio of the tissue compartment's mass or volume which is constituted of fat. The tissue compartment may be a muscle, such as a predetermined muscle. Such predetermined tissue compartment may be one of the muscles in the group of: the left or the right front thigh muscle, the left or the right back thigh muscle.

A concentration of fat infiltrated in at least one organ may provide a parameter value representing a percentage or ratio of the organ's mass or volume which is constituted of fat. The organ may be a predetermined organ. Such predetermined organ may for example be the liver, the heart or the pancreas.

A concentration of fat in bone marrow may provide a parameter value representing a percentage or ratio of the bone marrow in the individual's body which is constituted of fat.

The $BCP_I$ is compared with parameter values based on previous BCPs. This data could be parameter values in multiple stored BCPs, or parametric descriptions (such as intervals) extracted from parameter values in previously stored BCPs of other individuals.

In one embodiment, the values of volume of at least one tissue compartment and fat infiltration in at least one tissue compartment may be acquired for the same at least one tissue compartment.

A predetermined tissue compartment, such as a muscle, may be used for the volume and fat infiltration values. By using the same tissue compartment for both parameter values, certain information of the combined values may be determined. The volume or fat infiltration values may respectively not give a complete view of the status of the tissue compartment. A larger volume of a muscle may for instance manage a larger fat infiltration. Conclusions where both volume and fat infiltration values for the same tissue compartment are relevant may thereby be made. The predetermined tissue compartment may for instance be a thigh muscle.

In another embodiment, the step of determining the BCP may comprise determining a tissue compartment ratio by calculating the individual's body weight divided by the volume of said at least one tissue compartment.

The tissue compartment ratio may be a muscle ratio in the case that the volume of a muscle is used for the calculation. The muscle ratio may represent a ratio of mass per volume, for instance kilogram per liter.

In a further embodiment, the step of determining the BCP may comprise determining a fat ratio by calculating the sum of the amount of subcutaneous fat and the amount of visceral fat, divided by the sum of the amount of subcutaneous fat, the amount of visceral fat and the volume of said at least one tissue compartment. The fat ratio, FR, may further be expressed by the formulae:

$$FR = \frac{AF+VF}{AF+VF+TV},$$ where AF is the amount of subcutaneous fat, VF is the amount of visceral fat and TV is the volume of at least one tissue compartment.

The at least one tissue compartment may be a predetermined muscle, such as a thigh muscle. The fat ratio may represent a percentage of the amount of fat relative to the combination of the volume of the predetermined tissue compartment and the fat in the individual's body. The fat ratio may thereby provide an indicator of the amount of fat relative to the amount of tissue compartment volume in the individual's body. The tissue compartment volume may be the thigh muscle volume of the individual.

In one embodiment, the length and/or weight of the individual may be acquired, besides the at least two parameter values, and used in the determination of the BCP.

The length and/or weight of the individual may be used in combination with the at least two parameter values to determine the BCP. An extra dimension of the BCP and the evaluation of the individual's propensity for a certain health state may be provided.

In a further embodiment, the step of comparing the BCP of the individual may comprise comparing the BCP of the individual with either BCPs in a data base of other individuals to find a plurality of similar BCPs, or a predetermined parametric description, extracted from other individuals' BCPs, of the parameter values constituting the BCP of the individual.

The comparison of the BCP of the individual with data, such as parameter values, based on previously stored BCPs may be performed in two different ways. As a first option, the $BCP_I$ is compared to BCPs in a data base of other individuals to find a set of similar BCPs. The similar BCPs may be BCPs comprising at least the same parameters as the $BCP_I$, and wherein the param-

eter values of the $BCP_I$ and the stored BCPs are similar. By similar it may be meant that each parameter value of the stored BCPs is within a predetermined range of the corresponding $BCP_I$ parameter value. The predetermined range may be set for each evaluation based on characteristics of the data base. The predetermined range may also be selectable for each individual comparison.

Alternatively, the $BCP_I$ may be compared to a predetermined parametric description of the parameter values in the $BCP_I$. The parametric description may be connected to data that may be used for the evaluation of the individual's characteristics of at least one phenotype variable. The result of the comparison may depend on the $BCP_I$'s relation to the parametric description. The step of comparing the $BCP_I$ may comprise looking at parametric descriptions of the at least two parameters in combination. The known parametric description may relate to each parameter comprised in the determined BCP separately, or to the combination of parameters in the determined BCP. By comparing the $BCP_I$ with a known or predetermined parametric description, data connected to the parametric description may be used to evaluate the present individual's characteristics of a phenotype variable.

In one embodiment, the step of comparing the BCP of the individual with BCPs in a data base may comprise the step of selecting a group of stored BCPs from a data base of stored BCPs, the BCPs of the group having similar BCP as the individual's BCP.

A group of BCPs may thereby be selected, all which may be similar within a range to the $BCP_I$. For each of the parameter values used to determine the $BCP_I$ a range may be set around the value for the individual, wherein a parameter value of a stored BCP may be determined as similar to the corresponding parameter value of the $BCP_I$ if it is within said range. The range may be predetermined and may depend on which parameter it relates to. Known data connected to the BCPs in the group may then be used for evaluation of the individual's characteristics of at least one phenotype variable. Such known data may be medical history or outcomes of the individuals of the BCPs in the group.

In another embodiment, the parametric description may comprise at least one parametric interval for each of the at least two parameter values of the BCP, and the step of comparing may comprise comparing each of the parameter values of the individual to the at least one parametric interval.

The predetermined parametric intervals in the parametric description may be predetermined based on the BCPs of other individuals. Data connected to the individuals of the BCPs may have been used to determine the parametric intervals of interest in the comparison. Each of the parameter values in the $BCP_I$ may be compared to the parametric interval(s) for that parameter value. The comparisons for all parameter values of the $BCP_I$ may be combined to provide a basis for an evaluation of the individual's characteristics of a phenotype variable.

In a further embodiment, the step of comparing may comprise a step of categorizing the individual's characteristics of at least one phenotype variable into a group having predetermined phenotype variable characteristics.

When the $BCP_I$ is compared to a selected group of BCPs, or to a parametric description, the comparison may be made to categorize the individual's characteristics of a phenotype variable with predetermined phenotype variable characteristics. The comparison may provide information to select a predetermined category.

According to a second aspect of the invention, a computer program product is provided, which is configured to execute the method according to any of the embodiments above.

According to a third aspect of the invention, a readable computer medium comprising a computer program product according to the above is provided.

According to a fourth aspect of the invention, a system of providing a basis for an evaluation of an individual's characteristics of at least one phenotype variable is provided. The system comprises an input unit configured to receive or acquire at least two parameter values of the individual's body out of the group: amount of visceral fat, amount of subcutaneous fat, volume of at least one tissue compartment, concentration of fat infiltrated in at least one tissue compartment, concentration of fat infiltrated in at least one organ, and concentration of fat in bone marrow. The system further comprises a determination unit configured to determine a body composition profile for the individual based on the at least two parameter values in combination, and a comparing unit configured to compare the body composition profile of the individual with data which is based on previously stored body composition profiles of other individuals. The system may further in embodiments comprise units configured correspondingly as described for embodiments of the method above.

Brief Description of the Drawings

The invention will in the following be described in more detail with reference to the enclosed drawings, wherein:

Fig. 1 shows a flow chart of a method according to an embodiment of the invention;
Fig. 2 shows a schematic block diagram of a system according to an embodiment of the invention;
Figs. 3A-C show BCP diagrams according to embodiments of the present invention;
Figs. 4A-B show BCP diagrams according to embodiments of the present invention;
Fig. 5 shows BCP diagrams according to an embodiment of the present invention;
Fig. 6 shows a step of comparison according to an embodiment of the present invention; and

Fig. 7 shows example BCPs provided using an embodiment of the invention.

Description of Embodiments

The present invention will be described more fully hereinafter with reference to the accompanying drawings, in which preferred embodiments of the invention are shown. This invention may, however, be embodied in many different forms and should not be construed as limited to the embodiments set forth herein; rather, these embodiments are provided so that this disclosure will be thorough and complete, and will fully convey the scope of the invention to those skilled in the art. In the drawings, like numbers refer to like elements.

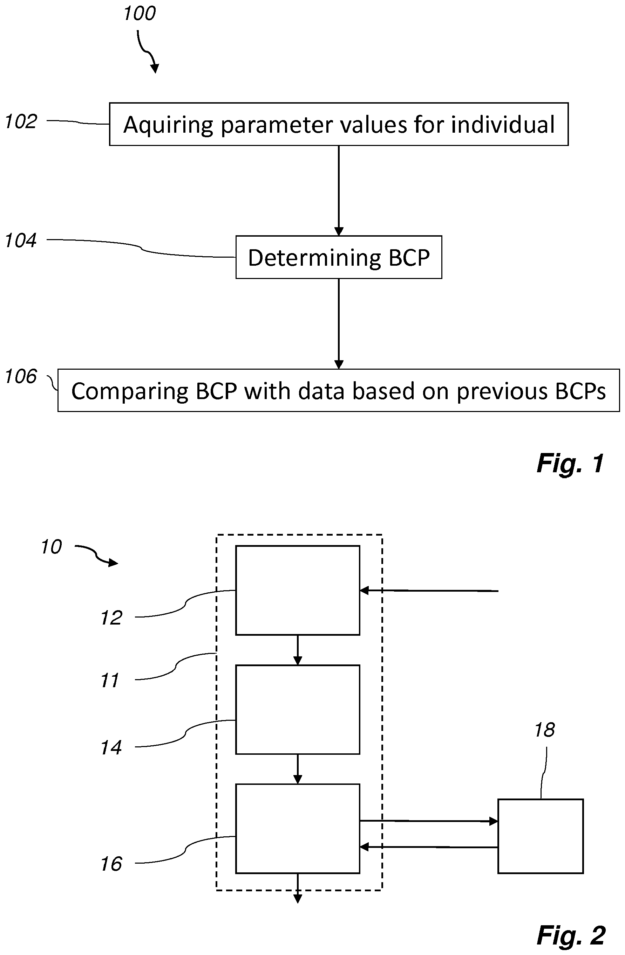

Fig. 1 illustrates a flowchart of a method 100 of providing a basis for an evaluation of an individual's characteristics of at least one phenotype variable according to an embodiment of the invention. The method 100 comprises a step of acquiring 102 at least two parameter values of the individual's body. The parameter values may be at least two out of the group of amount of visceral fat, amount of subcutaneous fat, volume of at least one tissue compartment, concentration of fat infiltrated in at least one tissue compartment, and concentration of fat in bone marrow.

The method 100 further comprises a step of determining 104 a body composition profile, $BCP_I$, for the individual using the at least two parameter values in combination. The two or more parameter values are used to provide, in combination, a representation of the individual's body.

Further, the method 100 comprises a step of comparing 106 the $BCP_I$ with data which is based on previously stored BCPs of other individuals.

Fig. 2 illustrates a system 10 configured to be used for providing a basis for an evaluation of an individual's characteristics of at least one phenotype variable. The system 10 comprises an input unit 12 configured to receive or acquire at least two parameter values of the individual's body. The parameter values could be manually entered into the input unit 12, or automatically received by the input unit 12 from a storing unit in which the parameter values have been stored, or from an acquisition means configured to provide the parameter values to the input unit 12. The system 10 further comprises a determination unit 14 configured to receive the parameter values from the input unit 12 and to determine the $BCP_I$ for the individual using the at least two received parameter values in combination. Further, the system 10 comprises a comparing unit 16 configured to receive the determined $BCP_I$ from the determination unit 14, and to compare the $BCP_I$ with data which is based on previously stored BCPs of other individuals. The previously stored BCPs are stored in a storing unit 18. The comparing unit 16 is communicatively connected to the storing unit 18. The storing unit 18 may be a stored database comprising BCPs and/or data (parameter values) generated from stored BCPs. The system 10 is in one embodiment provided by a processor unit 11, wherein processor unit 11 constitutes all of the input unit 12, the determining unit 14 and the comparing unit 16. The processor is communicatively connected to the storing unit 18.

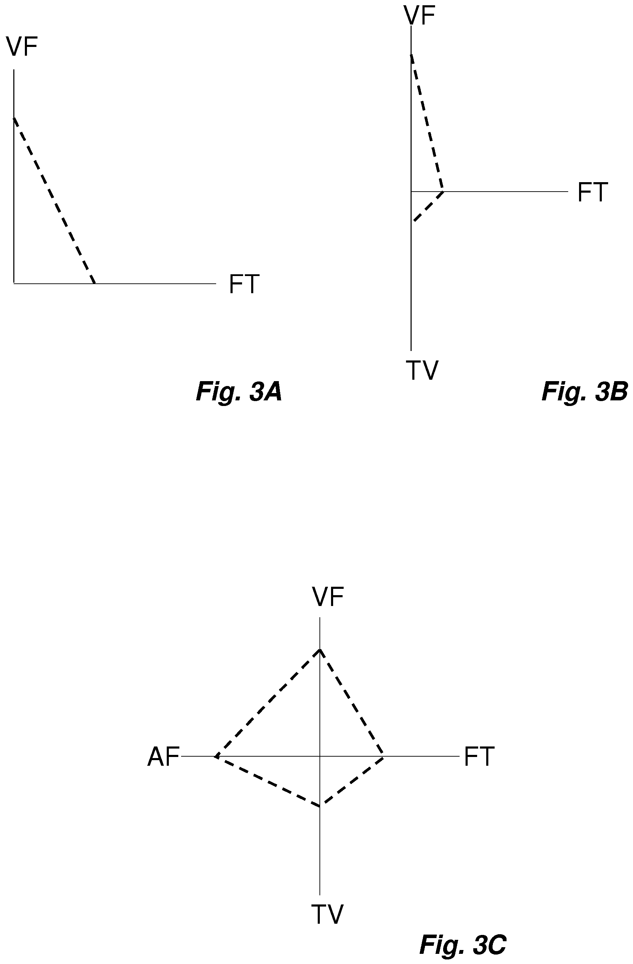

Figs. 3A-C illustrate embodiments of $BCP_I$s comprising two, three or four parameter values being visualized in a diagram. Fig. 3A illustrate an embodiment wherein the $BCP_I$ is determined using two parameter values:

amount of visceral fat, VF, and concentration of fat infiltrated in at least one tissue compartment, FT. A diagram is used to visualize the $BCP_I$. Fig. 3B illustrate an embodiment wherein the $BCP_I$ is determined using three parameter values: amount of visceral fat, VF, concentration of fat infiltrated in at least one tissue compartment, FT, and volume of at least one tissue compartment, TV. The tissue compartments in FT and TV are preferably one and the same. Fig. 3C illustrate an embodiment wherein the $BCP_I$ is determined using four parameter values: amount of visceral fat, VF, concentration of fat infiltrated in at least one tissue compartment, FT, volume of at least one tissue compartment, TV, and amount of abdominal subcutaneous fat, AF.

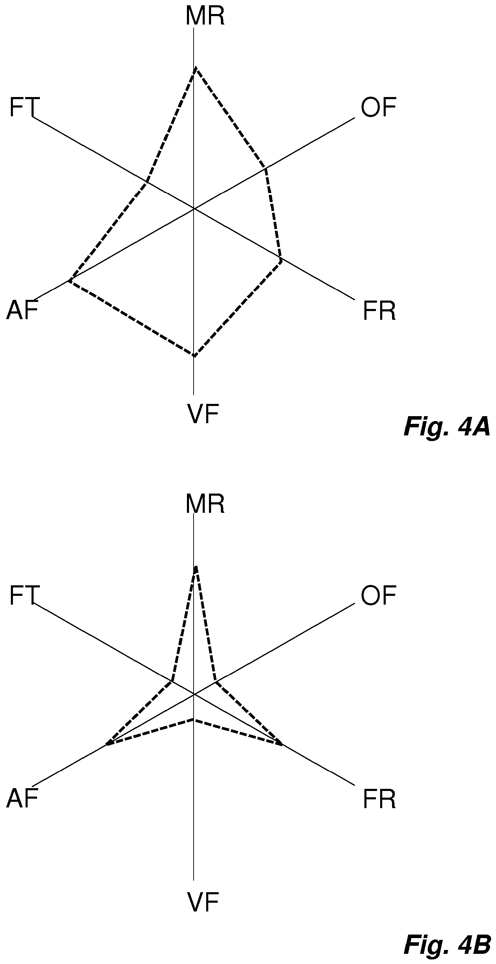

In one preferred embodiment, as illustrated in fig. 4, six parameter values are used to determine the $BCP_I$. The parameter values in the $BCP_I$ may comprise parameter values calculated out of one or more of the acquired parameter values and/or weight and length of the individual's body. In the illustrated embodiment, the six parameter values are provided by:

muscle ratio, MR, provided by a calculation of the body weight of the individual divided by the volume of a muscle. The at least one muscle may for instance be a front thigh muscle;
concentration of fat infiltrated in an organ, OF. Such organ may for instance be the liver;
fat ratio, FR, provided by a calculation of the sum of the amount of abdominal subcutaneous fat and the amount of visceral fat, divided by the sum of the amount of abdominal subcutaneous fat, the amount of visceral fat and the volume of a muscle. Such muscle may for instance be a front thigh muscle;
amount of visceral fat, VF;
amount of abdominal subcutaneous fat, AF; and
concentration of fat infiltrated in a muscle, FT. Such muscle may for instance be a front thigh muscle.

The abdominal subcutaneous fat in the embodiment above may in another embodiment be replaced by subcutaneous fat in another region of the body.

Fig. 4A illustrate a $BCP_I$ determined using the parameter values in combination. Another $BCP_I$ for another individual is illustrated in fig. 4B, using the same parameters, but which values in combination provide a different $BCP_I$. For instance, the individual represented in fig. 4A show a higher level of fat infiltrated in the liver, and a higher amount of visceral fat compared to the individual represented by the $BCP_I$ in fig. 4B.

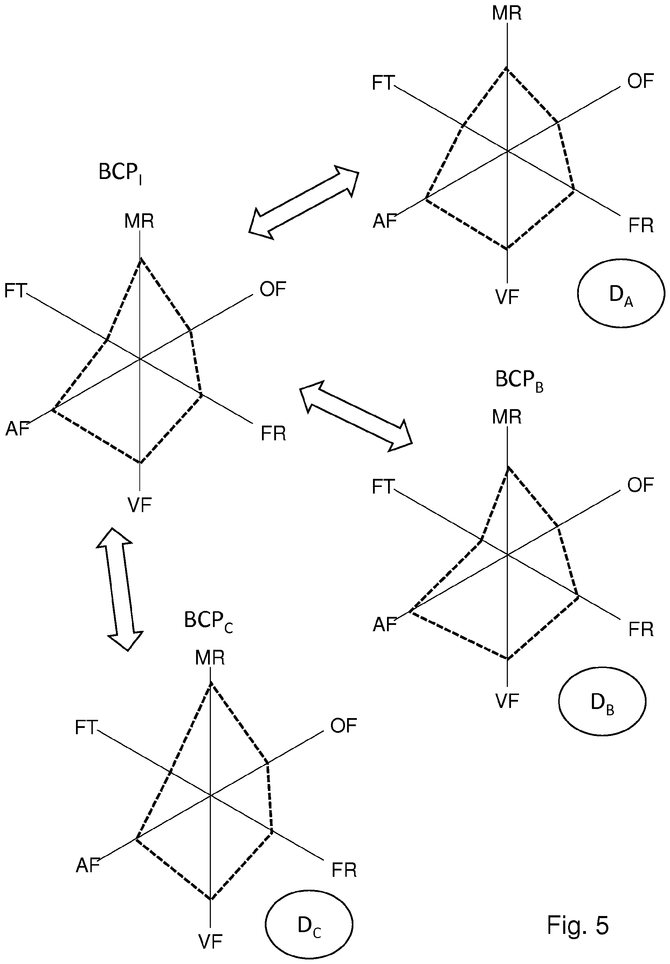

Fig. 5 illustrates a $BCP_I$ determined with the six parameters as discussed above. The $BCP_I$ is in the illustrated embodiment compared to three BCPs: $BCP_A$, $BCP_B$ and BCPc. The three of BCPs are selected out of a plurality of BCPs in a database. The selected BCPs are BCPs comprising values of at least the same parameters as the $BCP_I$. A selected BCP may comprise additional parameter values, not used when being compared with the $BCP_I$. Out of the plurality of BCPs in the database, BCPs are selected which have parameter values similar to the corresponding parameter values of the $BCP_I$. A parameter value of each selected BCP is categorized as similar when being within a range of the corresponding parameter value of the $BCP_I$. The range may in such case be predetermined, and may be individual for each parameter. The predetermined range may be set as a numerical value difference, plus and minus, of the parameter value of the $BCP_I$, or as a percentage difference, plus and minus, of the parameter value.

Each BCP in the database is a previously determined BCP for another individual. For each such individual, the database also comprises data D about the individual, such as health data over time, medical history and phenotype variables, in the illustrated embodiment provided as $D_A$-$D_C$. When the $BCP_I$ has been compared to the stored BCPs, and a selection of BCPs are determined as similar to the $BCP_I$, the data D about the individuals associated with the selected BCPs may be used to evaluate the characteristics of at least one phenotype variable for the individual of the $BCP_I$. Such characteristics of a phenotype variable may be a propensity for a certain health state such as a propensity for developing type 2 diabetes, the propensity for future cardiovascular events, the propensity of being metabolically healthy, or the propensity for developing a specific type of cancer.

The data $D_A$-$D_C$ associated with the selected BCPs, $BCP_A$-$BCP_C$, may for instance provide information about whether the individuals of the selected BCPs have had a cardiovascular disease. Depending on the data associated to all of the selected BCPs, an evaluation of the propensity for a future cardiovascular disease for the individual of the $BCP_I$ can be made. If prospective data is used, a risk for future disease may further be determined.

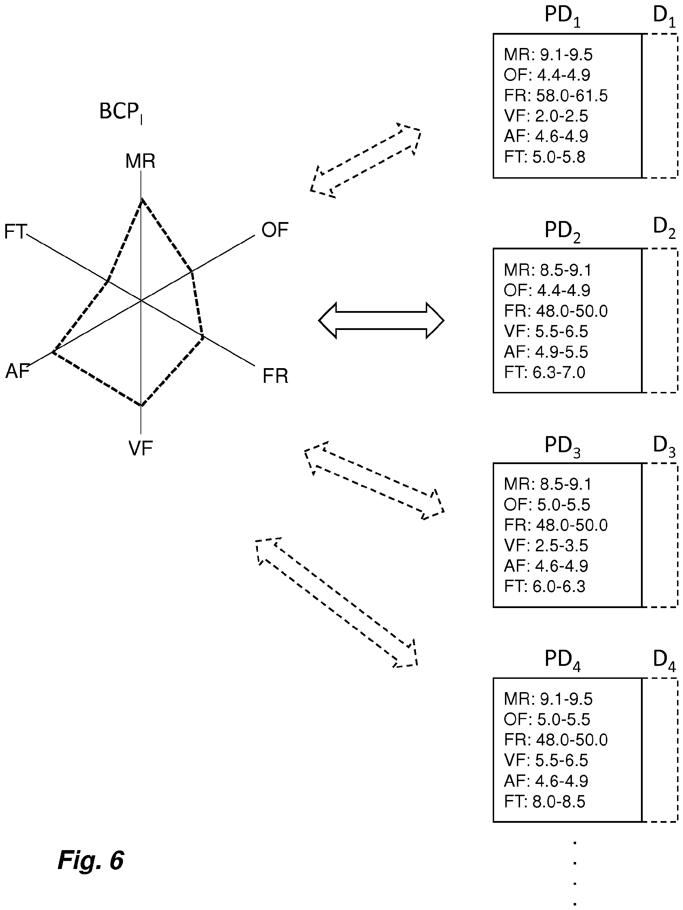

Fig. 6 illustrates an embodiment wherein the $BCP_I$ is compared to parametric descriptions PD of the parameters in the $BCP_I$, here exemplified by parametric descriptions $PD_1$-$PD_4$. Each parametric description PD comprises parametric intervals for each of the parameters in the $BCP_I$. The parametric descriptions may comprise additional parametric intervals for parameters not part of the present $BCP_I$. When comparing the $BCP_I$ to the available parametric descriptions PD, each parameter value in the $BCP_I$ is compared to the parametric intervals in each parametric description PD. Finally, a parametric description PD will be found when all the parameter values of the $BCP_I$ fall within the respective parametric interval in the same parametric description PD. The $BCP_I$ is thereby categorized to that parametric description.

Each parametric description PD is stored with data D, here exemplified by data $D_1$-$D_4$. The data is associated to the characteristics of at least one phenotype variable for an individual categorized to that specific parametric description. The categorization of the $BCP_I$ to $PD_2$ provides that data $D_2$ can be used for evaluation of the characteristics of at least one phenotype variable for the individual of the $BCP_I$. Such characteristics may be a propensity for a certain health state such as a propensity for developing type 2 diabetes or the propensity for future cardiovascular events.

The parametric descriptions and the respective data are pre-generated based on previously determined BCPs and the medical data of the individuals thereof. Each parametric description, and the parametric intervals thereof, may have been determined based on a plurality of BCPs and the medical history of the individuals.

The data $D_2$ of parametric description $PD_2$ may for instance provide that an individual whose $BCP_I$ has been categorized thereto has a 50% increased propensity to develop type 2 diabetes in the future compared to a reference $BCP_{ref}$ or reference parametric description $PD_{ref}$.

The determination of a BCP for an individual may be used as a tool that can effectively be used to further understanding of metabolic health. In an exemplary study, four principal findings were made. First, low ectopic fat, especially visceral fat and fat infiltrated in a tissue compartment, was positively associated with metabolic health and significantly higher values were found among subjects with metabolic diseases (coronary heart disease, CHD, and type 2 diabetes, T2D).

Second, subjects characterized as CHD and T2D exhibited different associations to BCP variables; Liver PDFF (proton density fat fraction) was found to have a positive association with T2D for all group comparisons and in the multivariable statistical modelling, whereas the association of liver PDFF with CHD was non-significant in the group comparison after matching on sex, age, and BMI, and negative when applying multivariable statistical modelling.

Third, these associations remained significant after adjusting the multivariable statistical models for sex, age, BMI, lifestyle factors, and statin treatment.

Lastly, within the same sex, age, and BMI groups, a variety of different individual BCPs were found, exhibiting different combinations of disease probabilities (CHD and T2D). Taken together, these findings suggest that there is more information in the BCP than what can be described by sex, age, lifestyle or generalized adiposity measured by BMI. They also show that different BCP phenotypes, or specific imbalances in fat accumulation, are linked to different diseases.

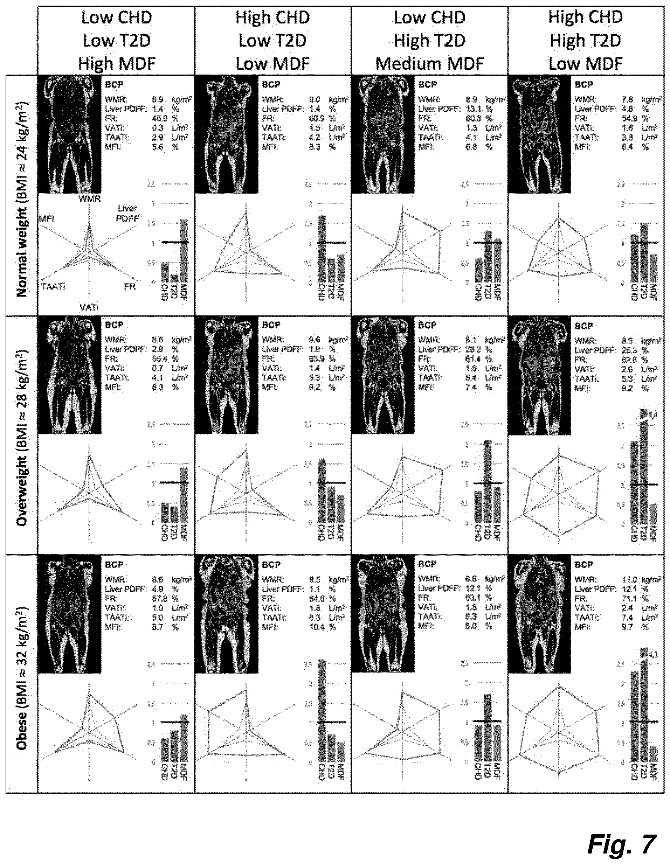

The investigation using body composition profiling has illustrated the need to individualize the description of metabolic health beyond what is achieved using BMI: the finding of obese subjects with lower disease probability comparing them to the normal weight population adds to the literature on healthy obesity. It was further strengthened by the comparison between a normal weight subject with inflated BCP (Fig. 7, top right subject) and obese male with a more star shaped BCP (Fig. 7, bottom left subject). The comparison yields a predicted probability for CHD with a factor 3 higher for the normal weight subject compared to the obese, a factor 2 higher for T2D, and a factor of about 0.5 lower for being metabolically disease free. Further, among subjects defined as normal weight, overweight, and obese, different BCPs were found, some of which associated with metabolic health, others with only CHD or T2D, and those exhibiting comorbid disease association. Individuals exhibiting high predicted probability for being metabolically disease free (Fig. 7, 1st column) had BCPs more similar to the metabolic disease free group, represented by the reference star (see Fig. 4B). Individuals exhibiting high predicted probability for CHD but low for T2D (Fig. 7, 2nd column) seemed to be characterized by high VAT and MFI, but low liver fat. Individuals exhibiting high predicted probability for T2D but low for CHD (Fig. 7, 3rd column) was characterized by high VAT and liver fat, but low MFI. And finally, individuals exhibiting comorbid disease association (Fig. 7, 4th column) was characterized by high VAT, liver fat, and MFI. With the identification of specific phenotypes defined by the BCP, and associated to different health state propensities, more targeted and effective disease treatments could be developed. Further, with an individualized description of a patient's metabolic disease status there is potential for highly individualized intervention plans.

In fig. 7, MRI scans of individuals in the example study is shown, wherein the scan illustrations are segmented to show visceral adipose tissue and abdominal adipose tissue. The BCPs with six parameter values are further shown. Further information is given by CHD = coronary heart disease; FR = fat ratio; MDF = metabolic disease free; MFI = muscle fat infiltration; PDFF = proton density fat fraction (concentration of fat infiltrated in organ); T2D = type 2 diabetes; TAATi = total abdominal adipose tissue index (abdominal subcutaneous fat); VATi = visceral adipose tissue index (visceral fat); WMR = weight-to-muscle ratio (muscle ratio).

In the drawings and specification, there have been disclosed preferred embodiments and examples of the invention and, although specific terms are employed, they are used in a generic and descriptive sense only and not for the purpose of limitation, the scope of the invention being set forth in the following claims.

## Claims

1. A method (100) of providing a basis for an evaluation of an individual's characteristics of at least one phenotypic variable, the method comprising the steps of:

   - acquiring (102) at least two of the following parameter values of the individual's body out of the group:

      - amount of visceral fat (VF);
      - amount of subcutaneous fat (AF);
      - volume of at least one tissue compartment (TV);
      - concentration of fat infiltrated in at least one tissue compartment (FT);
      - concentration of fat infiltrated in at least one organ (OF); and
      - concentration of fat in bone marrow;

   - determining (104) a body composition profile, BCP, for the individual using said at least two acquired parameter values in combination;
   - comparing (106) the BCP for the individual with parameter values which are based on previously stored BCPs of other individuals.

2. The method according to claim 1, wherein the values of volume of at least one tissue compartment (TV) and fat infiltration in at least one tissue compartment (FT) are acquired for the same at least one tissue compartment.

3. The method according to claims 1 or 2, wherein the step of determining the BCP comprises determining a tissue compartment ratio (MR) by calculating the individual's body weight divided by the volume of said at least one tissue compartment.

4. The method according to any of the preceding claims, wherein the step of determining the BCP comprises determining a fat ratio (FR) by calculating the sum of amount of subcutaneous fat and the amount of visceral fat, divided by the sum of the amount of subcutaneous fat plus the amount of visceral fat and the volume of said at least one tissue compartment.

5. The method according to any of the preceding claims, wherein the length and/or weight of the individual is acquired, besides the at least two parameter values, and used in the determination of the BCP.

6. The method according to any of the preceding claims, wherein the step of comparing (106) the BCP of the individual comprises comparing the BCP of the individual with either:

- BCPs (BCP$_A$, BCP$_B$, BCPc) in a data base of other individuals to find a plurality of similar BCPs, or
- a predetermined parametric description (PD$_1$-PD$_4$), extracted from other individuals' BCPs, of the parameter values constituting the BCP of the individual.

7. The method according to claim 6, wherein the step of comparing the BCP of the individual with BCPs (BCP$_A$, BCP$_B$, BCP$_C$) in a data base comprises the step of selecting a group of stored BCPs from a data base of stored BCPs, the BCPs of the group having similar BCP as the individual's BCP.

8. The method according to claim 6 or 7, wherein the parametric description (PD$_1$-PD$_4$) comprises at least one parametric interval for each of the at least two parameter values of the BCP, and wherein the step of comparing (106) comprises comparing each of the parameter values of the individual to the at least one parametric interval.

9. The method according to any of the preceding claims, wherein the step of comparing (106) comprises a step of categorizing the individual's characteristics of at least one phenotypic variable into a group having predetermined phenotype variable characteristics.

10. A computer program product configured to execute the method (100) of any of the preceding claims.

11. A readable computer medium comprising a computer program product according to claim 10.

12. A system (10) for providing a basis for an evaluation of an individual's characteristics of at least one phenotype variable, the system comprising:

an input unit (12) configured to receive or acquire at least two parameter values of the individual's body out of the group:

- amount of visceral fat;
- amount of subcutaneous fat;
- volume of at least one tissue compartment;
- concentration of fat infiltrated in at least one tissue compartment;
- concentration of fat infiltrated in at least one organ; and
- concentration of fat in bone marrow;

a determination unit (14) configured to determine a body composition profile (BCP$_I$) for the individual based on the at least two parameter values in combination; and
a comparing unit (16) configured to compare the body composition profile of the individual with data which is based on previously stored body composition profiles of other individuals.

*100*

102 — Aquiring parameter values for individual

104 — Determining BCP

106 — Comparing BCP with data based on previous BCPs

*Fig. 1*

*10*

12

11

14

18

16

*Fig. 2*

**Fig. 3A**

**Fig. 3B**

**Fig. 3C**

Fig. 4A

Fig. 4B

Fig. 5

Fig. 6

$PD_1$    $D_1$

MR: 9.1-9.5
OF: 4.4-4.9
FR: 58.0-61.5
VF: 2.0-2.5
AF: 4.6-4.9
FT: 5.0-5.8

$PD_2$    $D_2$

MR: 8.5-9.1
OF: 4.4-4.9
FR: 48.0-50.0
VF: 5.5-6.5
AF: 4.9-5.5
FT: 6.3-7.0

$PD_3$    $D_3$

MR: 8.5-9.1
OF: 5.0-5.5
FR: 48.0-50.0
VF: 2.5-3.5
AF: 4.6-4.9
FT: 6.0-6.3

$PD_4$    $D_4$

MR: 9.1-9.5
OF: 5.0-5.5
FR: 48.0-50.0
VF: 5.5-6.5
AF: 4.6-4.9
FT: 8.0-8.5

Fig. 7

Europäisches
Patentamt

European
Patent Office

Office européen
des brevets

# EUROPEAN SEARCH REPORT

Application Number

EP 17 19 8719

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2015/105651 A1 (ZHENG CHANG-JIANG [US]) 16 April 2015 (2015-04-16) <br> * paragraph [0004] - paragraph [0010] * <br> * paragraph [0017] - paragraph [0049] * <br> * paragraph [0224] * <br> ----- | 1-12 | INV. <br> G16H50/70 |
| | | | TECHNICAL FIELDS SEARCHED (IPC) <br><br> G16H |

The present search report has been drawn up for all claims

| Place of search | Date of completion of the search | Examiner |
|---|---|---|
| Munich | 21 March 2018 | Menschner, Philipp |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

........................................................................

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 17 19 8719

This annex lists the patent family members relating to the patent documents cited in the above-mentioned European search report.
The members are as contained in the European Patent Office EDP file on
The European Patent Office is in no way liable for these particulars which are merely given for the purpose of information.

21-03-2018

| Patent document cited in search report | Publication date | Patent family member(s) | Publication date |
|---|---|---|---|
| US 2015105651 A1 | 16-04-2015 | CN 106062754 A<br>EP 3058493 A1<br>JP 2017500675 A<br>US 2015105651 A1<br>WO 2015057965 A1 | 26-10-2016<br>24-08-2016<br>05-01-2017<br>16-04-2015<br>23-04-2015 |

EPO FORM P0459

For more details about this annex : see Official Journal of the European Patent Office, No. 12/82